# EUROPEAN PATENT SPECIFICATION

(11) **EP 3 071 294 B1**
(45) Date of publication and mention of the grant of the patent: **06.03.2019**
(21) Application number: 14864573.2
(22) Date of filing: 31.10.2014
(51) Int. Cl.: A61N 7/00, A61N 7/02

(54) **SKIN CANCER TREATMENT USING LOW INTENSITY ULTRASOUND**
HAUTKREBSBEHANDLUNG MITTELS ULTRASCHALL NIEDRIGER INTENSITÄT
TRAITEMENT D'UN CANCER DE LA PEAU UTILISANT DES ULTRASONS DE FAIBLE INTENSITÉ

(30) Priority: 22.11.2013 US 201361907748 P
(43) Date of publication of application: 28.09.2016
(73) Proprietor: Sonify Biosciences, LLC, Baltimore, Maryland 21201 (US)
(72) Inventor: BOER, Miriam Sara, Baltimore, Maryland 21201 (US); ROGERS, Daniel Jordan, Baltimore, Maryland 21201 (US)
(74) Representative: Kehoe, Laura Ellen
(86) International application number: PCT/US2014/063282
(87) International publication number: WO 2015/077006

(56) References cited:
- GB-A- 2 479 598
- GB-A- 2 479 598
- US-A1- 2002 040 199
- US-A1- 2003 083 536
- US-A1- 2008 027 359
- US-A1- 2008 255 478
- US-A1- 2008 255 478
- US-A1- 2010 063 565
- US-A1- 2011 269 693
- US-B2- 8 454 540

## Description

### TECHNICAL FIELD

The present invention relates generally to a system for skin cancer treatment using low intensity ultrasound.

### BACKGROUND

Skin cancer is the most common form of cancer in the United States. The prevalence of skin cancer is evident by the statistics that one in five Americans will develop skin cancer in the course of a lifetime.

The current treatment options for skin cancer include excisional surgery, radiation therapy, chemotherapy and immunotherapies, each of which has its own limitations. Excisional surgery is the most common method to treat skin cancer. However, because it is very difficult to remove all cancer cells, the likelihood of the cancer growing back can be high for certain types of cancers. Radiation therapy can result in adverse side effects such as chronic radiation dermatitis, skin atrophy and telangiectasia. Chemotherapy has adverse side effects as well, and its overall efficacy is low due to skin cancer's external presence. Immunotherapies are also plagued by serious side effects, including damages to other healthy organs, fever and severe tiredness. Accordingly, there is a strong need in the art to develop novel systems that can permit new skin cancer treatment.

US 2008/027359 A1 describes an ultrasonic device for thermal treatment of medical conditions near a skin surface, comprising an ultrasonic source adapted for depositing acoustical energy into a region of tissue near the skin surface. GB 2479598 describes a system for inducing hyperthermia and cavitation in a region of interest in a human or animal body. US 2003/083536 describes a system for lysing adipose tissue comprising a focussed ultrasonic energy director, directing focussed ultrasonic energy at a target volume in a region of a body containing adipose tissue, and a modulator cooperating with the energy director to produce a focussed ultrasonic energy so as to selectively lyse said adipose tissue in said target volume and generally not lyse non-adipose tissue in said target volume.

### SUMMARY

The invention relates to a system for skin cancer treatment in a subject using low intensity ultrasound. The system comprises an ultrasound transducer, a temperature sensing unit, and a control unit. The ultrasound transducer has a skin-contacting surface that is pre-formed in order to conform to the skin containing the cancer and the skin-contacting surface of the ultrasound transducer is produced by a process comprising a 3D laser scan of the surface of the skin containing the cancer. The temperature sensing unit is coupled to the ultrasound transducer, measures the temperature of the skin being treated with ultrasound, and sends temperature data to the control unit. The control unit is coupled to the ultrasound transducer and the temperature sensing unit, receives temperature data from the temperature sensing unit, and controls the ultrasound transducer as a function of the temperature data.

In some embodiments, the control unit controls ultrasound intensity at the focal zone produced by the ultrasound transducer as a function of the temperature data.

In some embodiments, the temperature sensing unit comprises one or more temperature sensors.

In some embodiments, the temperature sensor is a contact-type temperature sensor (e.g., a thermistor or a thermocouple) or a non-contact temperature sensor (e.g., an infrared sensor).

In some embodiments, the ultrasound transducer can produce ultrasound at a frequency between about 27 kHz and 2.2 MHz.

In some embodiments, the ultrasound transducer can produce ultrasound intensity at the focal zone between about 0.17 W/cm² and 5 W/cm².

In some embodiments, the subject is a mammal.

In some embodiments, the mammal is a human.

Also described is a method for skin cancer treatment in a subject. The method comprises (1) providing the system for applying low intensity ultrasound to the skin containing the cancer; (2) positioning the skin-contacting surface of an ultrasound transducer of the system onto the surface of the skin containing the cancer; and (3) exposing the skin containing the cancer to low intensity ultrasound. Skin cancers include, for example, basal cell carcinoma, squamous cell carcinoma, and melanoma. In some embodiments, the skin cancer is melanoma.

These and other capabilities of the invention, along with the invention itself, will be more fully understood after a review of the following figures, detailed description, and claims.

### BRIEF DESCRIPTION OF THE DRAWINGS

**Fig.** 1 is a schematic of a system according to some embodiments of the invention.
**Fig.** 2 is a schematic showing a portion of a system according to some embodiments of the invention.
**Fig. 3** is a flow chart describing an exemplary treatment for skin cancer using low intensity ultrasound according to some embodiments of the invention.

### DETAILED DESCRIPTION

The inventor has discovered, *inter alia,* a survival differential of cells exposed to low intensity ultrasound that correlated with cellular metabolic activities. Specifically, after ultrasound exposure, cells with lower metabolic activities tend to survive better than cells with higher metabolic activities. Metabolic activities for cells can be measured by assays such as the WST-1 cell proliferation assay.

Without wishing to be bound by theory, skin cancer cells typically have higher metabolic activity than normal skin cells as evident by the fact that skin cancer cells reproduce at a faster rate. Based on the inventor's discovery, skin cancer cells are more susceptible to low intensity ultrasound than normal skin cells. As used herein, the term "low intensity ultrasound" refers to ultrasound having frequency of 20 kHz or higher and an intensity at the focal zone of no more than 75 W/cm². As used herein, the term "susceptible" refers to higher percentage of death for skin cancer cells than normal skin cells when exposed to the same low intensity ultrasound. The percentage of death for skin cancer cells can be at least 10%, 20%, 30%, 40%, 50%, 60 %, 70%, 80%, or 90% higher than normal skin cells.

In order to treat skin cancer using ultrasound, a problem arises as how to effectively deliver ultrasound to the cancer for maximal therapeutic effects because skin has contours. Further, the contour can vary for the same subject depending on the location of the skin, or from subject to subject. Provided herein is an ultrasound system to overcome this challenge.

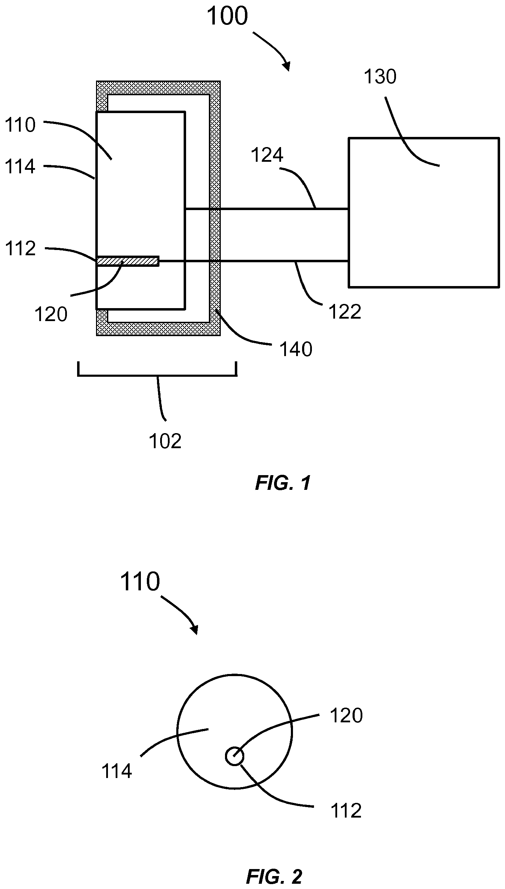

**Fig.** 1 is a schematic of a system 100 according to some embodiments of the invention. The system 100 comprises a control unit 130, a transducer unit 102 that includes an ultrasound transducer 110, a temperature sensing unit 120 and optionally a housing 140. The housing 140 can be made of molded plastic or similar materials. The ultrasound transducer 110 can include a skin-contacting surface 114. The temperature sensing unit 120 can be coupled to the ultrasound transducer 110 through a port 112 on the skin-contacting surface 114. The port 112 can be at any location on the skin-contacting surface 114. The port 112 can be a slot or a hole. In some embodiments, the port 112 can comprise electrical connections for the temperature sensing unit 120. The control unit 130 can be connected to the temperature sensing unit 120 and the ultrasound transducer 110 either through a physical connection (e.g., a wire or a cable) or wirelessly (e.g., Bluetooth or radio frequency). It should be noted that the first connection 122 between the control unit 130 and the temperature sensing unit 120 can be the same or different compared to the second connection 124 between the control unit 130 and the ultrasound transducer 110.

**Fig.** 2 is a schematic showing a portion of the system 100 that includes the skin-contacting surface 114 according to the invention.

Technologies of producing ultrasound are well known in the art and are not discussed in detail here. Generally, an ultrasound transducer converts one form of energy to ultrasonic energy. In some embodiments, the ultrasound transducer 110 comprises 1, 2, 3, 4, 5, 6, 7, 8, 9, 10, or more piezoelectric elements, and each piezoelectric element can convert electrical energy to ultrasound. The piezoelectric elements can be arranged in a pre-defined, irregular or regular pattern (e.g., rectangular, circular, octagonal, hexagonal, etc.). The piezoelectric element comprises a piezoelectric material that includes, but is not limited to, quartz, gallium orthophosphate, langasite, lead zirconate titanate, lead titanate, lead metaniobate, barium titanate, potassium niobate, lithium niobate, lithium tantalite, sodium tungstate, zinc oxide, Ba₂NaNb₅O₅, Pb₂KNb₅O₁₅, sodium potassium niobate, bismuth ferrite, sodium niobate, bismuth titanate, sodium bismuth titanate, polyvinylidene fluoride, or any combination thereof. In some embodiments, the ultrasound transducer 110 comprises a magnetostrictive material, and the magnetostrictive material can produce ultrasound when exposed to a magnetic field. Magnetostrictive materials include, but are not limited to, nickel, Fe-Al alloy, Fe-Ni alloy, Co-Ni alloy, Fe-Co alloy, Co-Fe-V alloy, CoFe₂O₄, NiFe₂O₄, or any combination thereof. The ultrasound transducer can further comprise components such as electrodes, backing, and wear plate. These components are common in ultrasound transducers and should be apparent to those skilled in the art.

The ultrasound transducer 110 can produce ultrasound having frequency in the range of 20 kHz to 100 MHz, 20 kHz to 50 MHz, 20 kHz to 25 MHz, 20 kHz to 10 MHz, 20 kHz to 5 MHz, 20 kHz to 2.5 MHz, or 27 kHz to 2.2 MHz. In some embodiments, the ultrasound frequency is about 27 kHz. In some embodiments, the ultrasound frequency is about 2.2 MHz. In some embodiments, the ultrasound frequency is not tunable. In some embodiments, the ultrasound frequency can be tuned. The ultrasound intensity at the focal zone can be changed, for example, by varying the magnitude of the voltage applied to the piezoelectric element. The ultrasound intensity at the focal zone is no more than 75 W/cm², 50 W/cm², 25 W/cm², 10 W/cm², 5 W/cm², 2 W/cm², 1 W/cm², or 0.5 W/cm². In some embodiments, the ultrasound intensity at the focal zone is in the range of about 0.1 to 10 W/cm², 0.1 to 5 W/cm², 0.5 to 10 W/cm², 0.5 to 5 W/cm², 1 to 10 W/cm², or 1 to 5 W/cm². In some embodiments, the ultrasound transducer 110 can produce continuous ultrasound. In some embodiments, the ultrasound transducer 110 can produce pulsed ultrasound.

During treatment, the skin-contacting surface 114 is in contact with the skin containing the cancer, while the ultrasound emanating from the ultrasound transducer 110 is focused onto the cancer. The skin-contacting surface 114 is pre-formed to conform to the skin containing the cancer on a subject. Without wishing to be bound by theory, the ability for an ultrasound transducer to conform to the skin allows the ultrasound transducer to deliver ultrasound more effectively to the site of interest, and thus resulting in enhanced therapeutic effects.

Prior to treatment, the skin-contacting surface 114 of the ultrasound transducer 110 is produced by a process that uses a 3D scan of the surface of the skin containing the cancer. The 3D scan can be acquired by 3D photography. According to the invention, the 3D scan is acquired by using a laser scanner. Systems and/or methods for 3D scanning are disclosed in, for example, WO2013126877, WO2004047009, WO2011135341, WO2012083967. Commercial products suitable for 3D skin scanning also exist, such as Antera 3D™ and Konica Minolta VIVID 910 3D laser scanner. Once the 3D scan is done, the data acquired (i.e., contour of skin containing the cancer) can then be used to select or fabricate a skin-contacting surface conforming to the skin being scanned. Methods of manufacture can include, but are not limited to, molding (e.g. injection molding), machining (e.g., including mechanical cutting, laser cutting and etching), extruding, embossing, solid free-form fabrication technologies (e.g., three dimensional printing and stereolithography), or any combination thereof. In some embodiments, the skin-contacting surface 114 of the ultrasound transducer 110 can be fabricated by three dimensional printing.

In some embodiments, the ultrasound transducer 110 can also be used to image the cancer and the surrounding tissue. As used herein, the term "image" or "imaging" refers to the act of producing a 2D or 3D picture of an object. Ultrasound has long been used in medical imaging, for example, in examining various organs (e.g., heart), and monitoring pregnancy.

The temperature sensing unit 120 measures the temperature of the skin being exposed to ultrasound and transmits temperature data to the control unit 130 through the first connection 122. The temperature measurement can be performed continuously or periodically (e.g., every 1, 2, 3, 4, 5, or more seconds). The temperature sensing unit 120 can comprise one or more temperature sensors. In some embodiments, the temperature sensor can be a contact-type temperature sensor (e.g., a thermistor or a thermocouple). In some embodiments, the temperature sensor can be a non-contact temperature sensor. In some embodiments, the non-contact temperature sensor can be an infrared sensor. In some embodiments, the infrared sensor can include a germanium sensor that is sensitive to electromagnetic radiation with wavelength between 8 um and 14 um. In some embodiments, the temperature sensing unit 120 can include two or more temperature sensors, whereby the two or more temperature sensors can perform differential temperature measurements. Differential temperature measurements measure the temperature difference between two points in a system. The accuracy of the temperature sensor should be better than 5 °C, 4 °C, 3 °C, 2 °C, 1.5 °C, 1 °C or less.

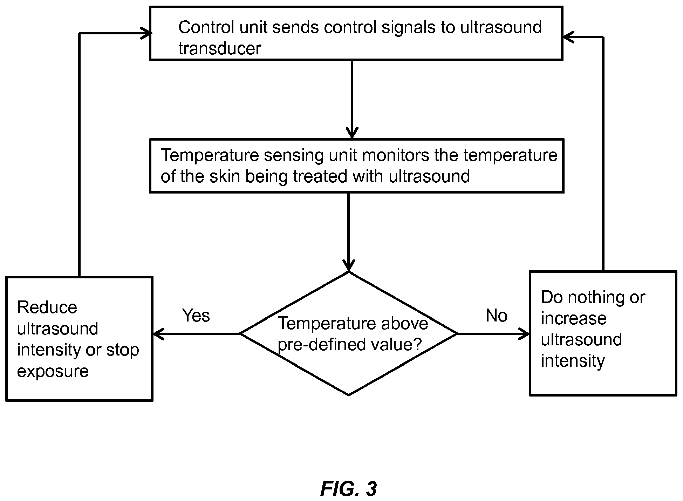

The control unit 130 has at least two main functions. First, the control unit 130 controls the ultrasound transducer 110 by determining the parameters for the ultrasound being produced (e.g., frequency and intensity). The control unit 130 can send control signals to the ultrasound transducer 110 through the second connection 124. Second, the control unit 130 receives temperature data from the temperature sensing unit 120, and based on the temperature data, decides whether to alter the parameters for the ultrasound. By way of example only, if the maximum temperature of the skin receiving ultrasound exposure is below a pre-defined value, the control unit 130 can either keep or increase the intensity. However, if the maximum temperature of the skin receiving ultrasound exposure exceeds a pre-defined value, the control unit 130 terminates the ultrasound exposure or reduces the ultrasound intensity at the focal zone in order to avoid skin damage or undue discomfort (Fig. 3). The pre-defined value can be between 40 °C and 100 °C, 40 °C and 90 °C, 40 °C and 80 °C, 40 °C and 70 °C, or 40 °C and 60 °C. In some embodiments, a physician/operator will monitor the temperature data and manually increase or reduce the ultrasound intensity at the focal zone, or terminate the ultrasound exposure. In some embodiments, the control unit can comprise computer programs including parameters that are set by a physician/operator. The computer programs can include treatment regimens, which determine the exposure duration and ultrasound characteristics (e.g., frequency and intensity), and change the ultrasound intensity based on the temperature data. The exposure regimens should be subject specific. In some embodiments, the control unit 130 can be coupled to a display module to display the temperature data, ultrasound parameters (e.g., frequency and intensity), and/or treatment regimens.

In some embodiments, the cancer is in the focal zone of the ultrasound. In some embodiments, the control unit 130 can control the size of the focal zone. Without wishing to be bound by theory, because the ultrasound intensity at the focal zone is determined by dividing the ultrasound energy per unit time at the focal zone by the area of the focal zone, for ultrasound of the same frequency, the larger the focal zone, the lower the intensity is at the focal zone. Therefore, changing the size of the focal zone can be used to change the ultrasound intensity at the focal zone. In some embodiments, the control unit 130 can control the position of the focal zone.

In some embodiments, the control unit can further comprise a storage module, whereby the temperature data and treatment regimens are stored. As used herein, the "storage module" is intended to include any suitable computing or processing apparatus or other device configured or adapted for storing data or information. Examples of storage devices suitable for use with the present invention include stand-alone computing apparatus; communications networks, including local area networks (LAN), wide area networks (WAN), Internet, Intranet, and Extranet; and local and distributed processing systems. Storage devices also include, but are not limited to: magnetic storage media, such as floppy discs, hard disc storage medium, and magnetic tape; optical storage media such as compact disc, DVD and Blu-ray TM discs; electronic storage media such as RAM, ROM, EPROM, EEPROM, solid state storage media and the like; general hard disks and hybrids of these categories such as magnetic/optical storage media.

The system described herein can be used for skin on any part of the body, including, but is not limited to, scalp, face, neck, arm, hand, torso, leg, or foot.

Also described herein is a method for skin cancer treatment in a subject using the system described herein. The method comprises (1) providing the system described herein; (2) positioning the skin-contacting surface of an ultrasound transducer of the system onto the surface of the skin containing the cancer; and (3) exposing the skin containing the cancer to low intensity ultrasound. Skin cancers include, for example, basal cell carcinoma, squamous cell carcinoma, and melanoma. In some embodiments, the skin cancer is melanoma.

In some embodiments, the subject who needs skin cancer treatment is a human or an animal. Usually the animal is a vertebrate such as, but is not limited to a primate, rodent, domestic animal or game animal. Primates include chimpanzees, cynomologous monkeys, spider monkeys, and macaques, e.g., Rhesus. Rodents include mice, rats, woodchucks, ferrets, rabbits and hamsters. Domestic and game animals include cows, horses, pigs, deer, bison, buffalo, feline species, e.g., domestic cat, canine species, e.g., dog, fox, wolf, avian species, e.g., chicken, emu, ostrich, and fish, e.g., trout, catfish and salmon. Patient or subject includes any subset of the foregoing, e.g., all of the above, but excluding one or more groups or species such as humans, primates or rodents. In certain embodiments of the aspects described herein, the subject is a mammal, e.g., a primate, e.g., a human. The terms, "patient" and "subject" are used interchangeably herein. A subject can be male or female. Additionally, a subject can be an infant or a child.

It should be understood that this invention is not limited to the particular methodology, protocols, and reagents, etc., described herein and as such may vary. The terminology used herein is for the purpose of describing particular embodiments only, and is not intended to limit the scope of the present invention, which is defined solely by the claims.

As used herein and in the claims, the singular forms include the plural reference and vice versa unless the context clearly indicates otherwise. Other than in the operating examples, or where otherwise indicated, all numbers expressing quantities of ingredients or reaction conditions used herein should be understood as modified in all instances by the term "about."

All patents and other publications identified are for the purpose of describing and disclosing, for example, the methodologies described in such publications that might be used in connection with the present invention. These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

Although any known methods, devices, and materials may be used in the practice or testing of the invention, the methods, devices, and materials in this regard are described herein.

### Some selected definitions

Unless stated otherwise, or implicit from context, the following terms and phrases include the meanings provided below. Unless explicitly stated otherwise, or apparent from context, the terms and phrases below do not exclude the meaning that the term or phrase has acquired in the art to which it pertains. The definitions are provided to aid in describing particular embodiments, and are not intended to limit the claimed invention, because the scope of the invention is limited only by the claims. Further, unless otherwise required by context, singular terms shall include pluralities and plural terms shall include the singular.

As used herein the term "comprising" or "comprises" is used in reference to compositions, methods, and respective component(s) thereof, that are useful to an embodiment, yet open to the inclusion of unspecified elements, whether useful or not.

The singular terms "a," "an," and "the" include plural referents unless context clearly indicates otherwise. Similarly, the word "or" is intended to include "and" unless the context clearly indicates otherwise.

Other than in the operating examples, or where otherwise indicated, all numbers expressing quantities of ingredients or reaction conditions used herein should be understood as modified in all instances by the term "about." The term "about" when used in connection with percentages may mean ±5% of the value being referred to. For example, about 100 means from 95 to 105.

Although methods and materials similar or equivalent to those described herein can be used in the practice or testing of this disclosure, suitable methods and materials are described below. The term "comprises" means "includes." The abbreviation, "e.g." is derived from the Latin exempli gratia, and is used herein to indicate a non-limiting example. Thus, the abbreviation "e.g." is synonymous with the term "for example."

As used herein, the terms "treat," "treatment," "treating," or "amelioration" refer to therapeutic treatments, wherein the object is to reverse, alleviate, ameliorate, inhibit, slow down or stop the progression or severity of a condition associated with a disease or disorder. The term "treating" includes reducing or alleviating at least one adverse effect or symptom of a condition, disease or disorder. Treatment is generally "effective" if one or more symptoms or clinical markers are reduced. Alternatively, treatment is "effective" if the progression of a disease is reduced or halted. That is, "treatment" includes not just the improvement of symptoms or markers, but also slowing of, progress or worsening of symptoms compared to what would be expected in the absence of treatment. Beneficial or desired clinical results include, but are not limited to, alleviation of one or more symptom(s), diminishment of extent of disease, stabilized (*i.e.,* not worsening) state of disease, delay or slowing of disease progression, amelioration or palliation of the disease state, remission (whether partial or total), and/or decreased morbidity or mortality. The term "treatment" of a disease also includes providing relief from the symptoms or side-effects of the disease (including palliative treatment).

As used herein, the term "focal zone" is defined as an area where the ultrasound energy converges. Typically, the focal zone is the area at which the ultrasound beam is at its narrowest and the ultrasound intensity is the greatest. The ultrasound intensity at the focal zone is determined by dividing the ultrasound energy per unit time at the focal zone by the area of the focal zone. For ultrasound of the same frequency, a user can control the area of the focal zone by varying how tightly the ultrasound is focused.

As used herein, the term "duration of exposure" refers to the amount of time when skin is continuously exposed to ultrasound.

Although preferred embodiments have been depicted and described in detail herein, it will be apparent to those skilled in the relevant art that various modifications, additions, substitutions, and the like can be made without departing from the invention and these are therefore considered to be within the scope of the invention as defined in the claims which follow. Further, to the extent not already indicated, it will be understood by those of ordinary skill in the art that any one of the various embodiments herein described and illustrated can be further modified to incorporate features shown in any of the other embodiments disclosed herein.

All patents and other publications identified in the specification and examples are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

## Claims

1. A system (100) for skin cancer treatment in a subject using low intensity ultrasound, comprising:
(a) an ultrasound transducer (110), wherein the ultrasound transducer has a skin-contacting surface (114) pre-formed to conform to the skin containing the cancer;
(b) a temperature sensing unit (120) coupled to the ultrasound transducer, wherein the temperature sensing unit measures the temperature of the skin being treated with ultrasound; and
(c) a control unit (130) coupled to the ultrasound transducer and the temperature sensing unit, wherein the control unit receives temperature data from the temperature sensing unit, and wherein the control unit controls the ultrasound transducer as a function of the temperature data;
**characterized in that**:
the skin-contacting surface of the ultrasound transducer is produced by a process comprising a 3D laser scan of the surface of the skin containing the cancer.

2. The system of claim 1, wherein the control unit controls ultrasound intensity at the focal zone produced by the ultrasound transducer as a function of the temperature data.

3. The system of claim 1 or 2, wherein the temperature sensing unit comprises one or more temperature sensors.

4. The system of claim 3, wherein the temperature sensor is a contact-type temperature sensor.

5. The system of claim 4, wherein the contact-type temperature sensor is a thermistor or a thermocouple.

6. The system of claim 3, wherein the temperature sensor is a non-contact temperature sensor.

7. The system of claim 6, wherein the non-contact temperature sensor is an infrared sensor.

8. The system of any of claims 1 to 7, wherein the ultrasound transducer can produce ultrasound at a frequency between about 27 kHz and 2.2 MHz.

9. The system of any of claims 1 to 8, wherein the ultrasound transducer can produce ultrasound intensity at the focal zone between about 0.17 W/cm² and 5 W/cm².

10. The system of any of claims 1 to 9, wherein the subject is a mammal.

11. The system of claim 10, wherein the mammal is a human.

## Patentansprüche

1. System (100) zur Behandlung von Hautkrebs in einem Subjekt unter Verwendung von Ultraschall mit niedriger Intensität, Folgendes umfassend:
(a) einen Ultraschallwandler (110), wobei der Ultraschallwandler eine hautberührende Oberfläche (114) aufweist, die vorgeformt ist, um sich der den Krebs enthaltenden Haut anzupassen;
(b) eine Temperaturmesseinheit (120), die an den Ultraschallwandler gekoppelt ist, wobei die Temperaturmesseinheit die Temperatur der Haut, die mit dem Ultraschall behandelt wird, misst; und
(c) eine Steuereinheit (130), die an den Ultraschallwandler und an die Temperaturmesseinheit gekoppelt ist, wobei die Steuereinheit Temperaturdaten von der Temperaturmesseinheit empfängt und wobei die Steuereinheit den Ultraschallwandler abhängig von den Temperaturdaten steuert;
**dadurch gekennzeichnet, dass**:
die hautberührende Oberfläche des Ultraschallwandlers durch einen Vorgang hergestellt wird, der einen 3D-Laserscan der Oberfläche der den Krebs enthaltenden Haut umfasst.

2. System nach Anspruch 1, wobei die Steuereinheit die Ultraschallintensität in der Fokalzone steuert, die durch den Ultraschallwandler abhängig von den Temperaturdaten hergestellt wird.

3. System nach Anspruch 1 oder 2, wobei die Temperaturmesseinheit einen oder mehrere Temperatursensoren umfasst.

4. System nach Anspruch 3, wobei der Temperatursensor ein Temperatursensor des Kontakttyps ist.

5. System nach Anspruch 4, wobei der Temperatursensor des Kontakttyps ein Thermistor oder ein Thermoelement ist.

6. System nach Anspruch 3, wobei der Temperatursensor ein kontaktloser Temperatursensor ist.

7. System nach Anspruch 6, wobei der kontaktlose Temperatursensor ein Infrarotsensor ist.

8. System nach einem der Ansprüche 1 bis 7, wobei der Ultraschallwandler einen Ultraschall mit einer Frequenz zwischen 27 kHz und 2,2 MHz herstellen kann.

9. System nach einem der Ansprüche 1 bis 8, wobei der Ultraschallwandler eine Ultraschallintensität in der Fokalzone zwischen etwa 0,17 W/cm² und 5 W/cm² herstellen kann.

10. System nach einem der Ansprüche 1 bis 9, wobei das Subjekt ein Säugetier ist.

11. System nach Anspruch 10, wobei das Säugetier ein Mensch ist.

## Revendications

1. Système (100) pour le traitement du cancer de la peau chez un sujet en utilisant des ultrasons de faible intensité, comprenant :
(a) un émetteur-récepteur d'ultrasons (110), l'émetteur-récepteur d'ultrasons comportant une surface de contact avec la peau (114) préformée pour se conformer à la peau contenant le cancer ;
(b) une unité de détection de température (120) couplée à l'émetteur-récepteur d'ultrasons, l'unité de détection de température mesurant la température de la peau traitée par ultrasons ; et
(c) une unité de contrôle (130) couplée à l'émetteur-récepteur d'ultrasons et à l'unité de détection de température, l'unité de contrôle recevant des données de température de l'unité de détection de température, et l'unité de contrôle contrôlant l'émetteur-récepteur d'ultrasons en fonction des données de température ;
**caractérisé en ce que** :
la surface de contact avec la peau de l'émetteur-récepteur d'ultrasons est produite par un procédé comprenant un balayage laser 3D de la surface de la peau contenant le cancer.

2. Système selon la revendication 1, dans lequel l'unité de contrôle contrôle l'intensité des ultrasons au niveau de la zone focale, produits par l'émetteur-récepteur d'ultrasons, en fonction des données de température.

3. Système selon la revendication 1 ou 2, dans lequel l'unité de détection de température comprend un ou plusieurs capteurs de température.

4. Système selon la revendication 3, dans lequel le capteur de température est un capteur de température de type à contact.

5. Système selon la revendication 4, dans lequel le capteur de température à contact est un thermistor ou un thermocouple.

6. Système selon la revendication 3, dans lequel le capteur de température est un capteur de température sans contact.

7. Système selon la revendication 6, dans lequel le capteur de température sans contact est un capteur infrarouge.

8. Système selon l'une quelconque des revendications 1 à 7, dans lequel l'émetteur-récepteur d'ultrasons peut produire des ultrasons à une fréquence entre environ 27 kHz et 2,2 MHz.

9. Système selon l'une quelconque des revendications 1 à 8, dans lequel l'émetteur-récepteur d'ultrasons peut produire des intensités d'ultrasons au niveau de la zone focale entre environ 0,17 W/cm² et 5 W/cm².

10. Système selon l'une quelconque des revendications 1 à 9, dans lequel le sujet est un mammifère.

11. Système selon la revendication 10, dans lequel le mammifère est un être humain.
